(19) Europäisches Patentamt
European Patent Office
Office européen des brevets

(11) Veröffentlichungsnummer : **0 033 114**
**B1**

(12) # EUROPÄISCHE PATENTSCHRIFT

(45) Veröffentlichungstag der Patentschrift : **24.08.83**

(21) Anmeldenummer : **81100361.5**

(22) Anmeldetag : **19.01.81**

(51) Int. Cl.³ : **C 07 C157/12**, C 07 D307/24, C 07 D307/68, C 07 D309/08, C 07 D333/38, C 07 D261/18, C 07 D263/34, A 01 N 47/34// C07C125/08

(54) **Fungizide substituierte Thioharnstoffe, ihre Herstellung und Verwendung zur Bekämpfung von Fungi und Mittel dafür.**

(30) Priorität : **28.01.80 DE 3002925**

(43) Veröffentlichungstag der Anmeldung : **05.08.81 Patentblatt 81/31**

(45) Bekanntmachung des Hinweises auf die Patenterteilung : **24.08.83 Patentblatt 83/34**

(84) Benannte Vertragsstaaten : **AT BE CH DE FR GB IT LI NL SE**

(56) Entgegenhaltungen : **DE A 2 805 525**

(73) Patentinhaber : **BASF Aktiengesellschaft**
**Carl-Bosch-Strasse 38**
**D-6700 Ludwigshafen (DE)**

(72) Erfinder : **Eicken, Karl, Dr.**
**Waldstrasse 63**
**D-6706 Wachenheim (DE)**
Erfinder : **Pommer, Ernst-Heinrich, Dr.**
**Berliner Platz 7**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 033 114 B1

# Fungizide substituierte Thioharnstoffe, ihre Herstellung und Verwendung zur Bekämpfung von Fungi und Mittel dafür

Die vorliegende Erfindung betrifft neue substituierte Thioharnstoffe, ein Verfahren zu ihrer Herstellung, deren Verwendung als Fungizide, fungizide Mischungen, die diese Wirkstoffe enthalten, Verfahren zur Herstellung solcher fungiziden Mischungen sowie Verfahren zur Bekämpfung von Schadpilzen mit diesen Fungiziden oder fungiziden Mischungen, die diese Verbindungen enthalten.

Es ist bereits bekannt, daß 2-Cyano-N-(ethylaminocarbonyl)-2-(methoxyimino)-acetamid eine fungizide Wirkung gegenüber niederen Pilzen hat (US-PS 3 954 992). Es ist ferner bekannt, das N-Trichlormethylthio-tetrahydrophthalimid zur Bekämpfung von Pilzen zu verwenden (Chemical Week, June 21, 1972, Seite 46).

Es wurde gefunden, daß substituierte Thioharnstoffe der Formel I

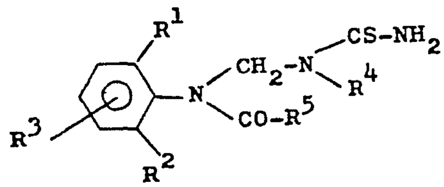

(I)

in welcher

$R^1$ $C_1$-$C_3$-Alkyl,

$R^2$ und/oder $R^3$ Wasserstoff, $C_1$-$C_3$-Alkyl oder Halogen,

$R^4$ $C_1$-$C_3$-Alkoxycarbonyl, $C_3$-$C_4$-Alkenoxycarbonyl, $C_1$-$C_2$-Alkylthiocarbonyl,

$R^5$ ein gegebenenfalls durch $C_1$-$C_3$-Alkoxy oder $C_1$-$C_3$-Alkylthio substituiertes $C_1$-$C_6$-Alkyl, ein $C_2$-$C_4$-Alkenyl, ein $C_3$-$C_6$-Cycloalkyl, einen gegebenenfalls durch Methyl oder Halogen substituierten heterocyclischen Rest bedeutet, der ein Sauerstoffatom oder ein Schwefelatom oder ein Sauerstoffatom und ein Stickstoffatom enthält, ferner ein Phenyl oder ein durch ein bis drei gleiche oder verschiedene Substituenten aus der Gruppe $C_1$-$C_3$-Alkyl, Fluor, Chlor, Brom, Trifluormethyl substituiertes Phenyl bedeutet, eine gute fungizide Wirkung gegenüber phytopathogenen Pilzen haben.

Unter Alkyl und Alkylrest einer Alkoxygruppe bei den Resten $R^1$, $R^2$, $R^3$, $R^4$ und $R^5$ sind je nach Zahl der genannten Kohlenstoffatome beispielsweise folgende Gruppen zu verstehen ; Methyl, Ethyl, N-Propyl, iso-Propyl, n-, iso-, sec.- oder tert.-Butyl, Pentyl oder Hexyl und ihre Isomeren.

Als Alkenyl sind vor allem Vinyl, Allyl und Methallyl zu nennen.

Als Cycloalkyl ist vor allem Cyclopropyl, Cyclobutyl, Cyclopentyl und Cyclohexyl zu nennen.

Unter Halogen sind beispielsweise Fluor, Chlor oder Brom zu verstehen. Als heterocyclischer Rest in der Bedeutung für $R^5$ sind vorzugsweise 5- bis 6-gliedrige Reste zu nennen, beispielsweise Furan, Thiophen, Oxazol, Isoxazol, Thiazol, Tetrahydrofuran, 2,3-Dihydropyran oder Tetrahydropyran, welche gegebenenfalls durch Methyl und/oder Halogen substituiert sein können.

Als substituiertes Phenyl für $R^5$ gelten vor allem beispielsweise monosubstituierte Phenyle wie Fluorphenyl, Chlorphenyl, Bromphenyl, Trifluormethylphenyl, Tolyl ; ferner disubstituierte Phenyle wie Dichlorphenyl, Difluorphenyl, Fluorchlorphenyl, Methylfluorphenyl, Methylchlorphenyl, Methylbromphenyl, Methyltrifluormethylphenyl, Chlortrifluormethylphenyl.

Es wurde ferner gefunden, daß man die substituierten Thioharnstoffe der Formel I erhält, wenn man substituierte Cyanamide der Formel II

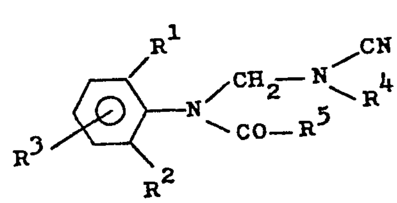

(II)

in welcher $R^1$, $R^2$, $R^3$, $R^4$ und $R^5$ die oben angegebene Bedeutung haben, mit Schwefelwasserstoff umsetzt.

Die Umsetzung von Alkyl-dicyanamiden mit Schwefelwasserstoff ist bekannt (Synthesis *1978*, 775).

Die Umsetzung der substituierten Cyanamide der Formel II mit Schwefelwasserstoff kann in Anwesenheit oder Abwesenheit von Lösungsmitteln durchgeführt werden, gegenüber denen die Reaktionspartner inert sind. Als Lösungsmittel kommen vor allem Ether — wie Diethylether, tert.-Butylmethylether, Glykoldimethylether, Tetrahydrofuran, Dioxan — Ester wie Essigester, N,N-Dialkylamide wie Dimethylformamid und Gemische dieser Lösungsmittel in Frage. Die Reaktionstemperaturen liegen zwischen − 20 °C und 150 °C, vorzugsweise zwischen 20 °C und 100 °C. Zur Beschleunigung der Umsetzung mit Schwefelwasserstoff kann es vorteilhaft sein, tertiäre organische Basen als Katalysatoren

in Mengen von 1-30 Molprozent, bezogen auf eingesetztes Cyanamid der Formel II zuzusetzen. Als tertiäre organische Basen kommen vor allem tertiäre Amine, z. B. Triethylamin, Pyridin und substituierte Pyridine in Betracht.

Zur Herstellung der neuen Thioharnstoffe setzt man auf 1 Mol substituiertes Cyanamid der Formel II mindestens 1 Mol Schwefelwasserstoff ein. Die Reaktion kann wahlweise unter Druck oder bei Normaldruck durchgeführt werden. Nicht verbrauchter Schwefelwasserstoff kann in die Reaktion zurückgeführt werden.

Zur Isolierung der Verbindungen der Formel I wird nach Entfernen von gelöstem Schwefelwasserstoff durch Anlegen von Unterdruck oder Ausblasen mit Inertgas das Lösungsmittel abdestilliert. Das erhaltene Produkt der Formel I ist in vielen Fällen rein, wenn nötig kann es durch Umkristallisieren oder durch Chromatographie an Kieselgel weiter gereinigt werden. Bei Verwendung mit Wasser mischbarer Lösungsmittel, z. B. Tetrahydrofuran, Dioxan, lassen sich in manchen Fällen die Verbindungen der Formel I durch Verdünnen mit Wasser ausfällen.

Die Herstellung der als Ausgangsverbindungen dienenden substituierten Cyanamide der Formel II erfolgt durch Umsetzungen von einem N-Halogenmethylanilid der Formel III

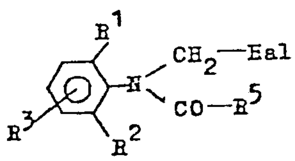

(III)

in welcher $R^1$, $R^2$, $R^3$ und $R^5$ die oben angegebene Bedeutung haben und Hal Halogen, insbesondere Chlor oder Brom bedeutet, mit einem Cyanamid der Formel IV

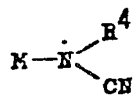

(IV)

in welcher $R^4$ die oben angegebene Bedeutung hat und M Wasserstoff, ein Alkalimetall oder ein Tetraalkylammonium-Rest bedeutet, gegebenenfalls in Gegenwart eines Säurebindemittels und gegebenenfalls in Gegenwart eines gegenüber den Reaktionspartnern inerten Lösungsmittels.

Die als Vorprodukte verwendeten N-Halogenmethylanilide der Formel III sind teilweise bekannt (US-PS 3 637 847) oder können in an sich bekannter Weise durch Umsetzung von entsprechend substituierten Phenylazomethinen mit Säurechloriden der Formel $R^5$—CO—Hal hergestellt werden, wobei $R^5$ die oben genannten Bedeutungen hat und Hal Halogen bedeutet.

Cyanamid-Verbindungen der Formel IV sind bekannte Verbindungen, beispielsweise : Cyancarbaminsäureester (DE-PS 24 74 453, DE-OS 17 95 849) Cyancarbaminsäurethiolester (Yuki Cosei Kagaku Kyokai Shi 1971, *29* (1) 67 (CA : *74,* 140877 u)) und Dicyanimid (Liebigs Ann. Chem. *427,* 1 1922 ; J. Chem. Soc. C, *1970,* 875).

Die folgenden Beispiele erläutern die Herstellung der neuen Thioharnstoff-Verbindungen der Formel I sowie die Herstellung der Ausgangsmaterialien der Formel II.

In den Beispielen verhalten sich Gewichtsteile zu Volumenteilen wie Kilogramm zu Liter.

A. Herstellung der Ausgangsmaterialien :

Zu einer Lösung von 12,2 Gewichtsteilen des Natriumsalzes des Cyancarbaminsäuremethylesters und 1 Teil Benzyl-triäthylammoniumchlorid in 30 Volumenteilen Wasser tropfte man bei 20 bis 25 °C eine Lösung von 26,4 Gewichtsteilen N-(Chlormethyl)-furan-2-carbonsäure-2',6'-dimethylanilid in 70 Volumenteilen Methylenchlorid unter intensiver Vermischung der Phasen. Nach 6-stündiger Durchmischung wurde die organische Phase abgetrennt, dreimal mit Wasser gewaschen und über Natriumsulfat getrocknet. Nach dem Verdampfen des Lösungsmittels isolierte man 29,0 Gewichtsteile N-Methoxycarbonyl-N-(N'-furyl-2-carbonyl-N'-2',6'-dimethylanilinomethyl)-cyanamid, Fp 96-98 °C (Methanol).

Analog erhält man folgende Verbindungen der Formel II :

| $R^1$ | $R^2$ | $R^3$ | $R^4$ | $R^5$ | Fp(°C) bzw. $n_D^{20}$ |
|---|---|---|---|---|---|
| $CH_3$ | $CH_3$ | H | $CO_2CH_3$ | $CH_3$ | 112-114 |
| " | " | " | " | 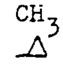 | 106 |
| " | " | " | " | $CH=CH_2$ | 92 |

(Fortsetzung)

| $R^1$ | $R^2$ | $R^3$ | $R^4$ | $R^5$ | Fp(°C) bzw. $n_D^{20}$ |
|---|---|---|---|---|---|
| " | " | " | " | $C(=CH_2)CH_3$ | 90 |
| " | " | " | " | $CH_2OCH_3$ | 74-76 |
| " | " | " | $CO_2C_2H_5$ | " | 86 |
| " | " | " | $CO_2CH_3$ | $CH_2OC_2H_5$ | 78 |
| " | " | " | $CO-SCH_3$ | $CH_2OCH_3$ | 118 |
| " | " | 3-$CH_3$ | $CO_2CH_3$ | " | Öl |
| " | " | H | " | 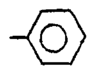 | 136 |
| " | " | " | " | 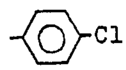 | 161 |
| $CH_3$ | $CH_3$ | H | $CO_2CH_3$ | 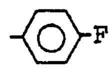 | 145 |
| " | " | " | " | 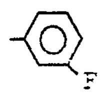 | 136 |
| " | " | " | " | 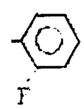 | 117 |
| " | " | " | " | 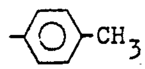 | 117-119 |
| " | " | " | " | 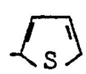 | 115-116 |
| " | " | " | " | 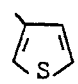 | 121 |
| " | " | " | " | 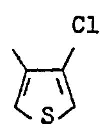 | 130-132 |
| " | $C_2H_5$ | " | " | 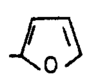 | Öl |
| " | $CH_3$ | 3-$CH_3$ | " | " | Öl |
| " | " | H | " | 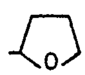 | |
| " | " | " | " | 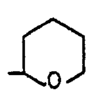 | |
| " | " | " | " | 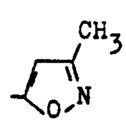 | 106-108 |

(Fortsetzung)

| $R^1$ | $R^2$ | $R^3$ | $R^4$ | $R^5$ | $Fp(^\circ C)$ bzw. $n_D^{20}$ |
|---|---|---|---|---|---|
| $CH_3$ | $CH_3$ | H | $CO_2CH_3$ | 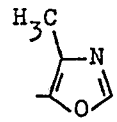 | 122 |
| " | " | " | " | 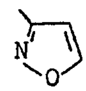 | 99 |
| " | " | " | " | 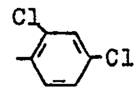 | 90 |
| " | " | " | " | 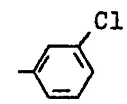 | Öl |
| " | " | " | " | 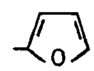 | 96-98 |

B. Herstellung der Endprodukte

Beispiel 1

In eine Lösung von 20,0 Gewichtsteilen N-Methoxycarbonyl-N-(N'-methoxyacetyl-N'-2',6'-dimethylanilinomethyl)-cyanamid und einem Volumenteil Triethylamin in 50 Volumenteilen Tetrahydrofuran wurde 1,5 Stunden bei 22 °C Schwefelwasserstoff eingeleitet, wobei eine schwach exotherme Reaktion (28 °C) eintrat. Nach vollständiger Umsetzung der Ausgangsverbindung wurde der überschüssige Schwefelwasserstoff mit Stickstoff ausgeblasen und nach Zutropfen von 200 Volumenteilen Wasser 18,6 Gewichtsteile N-Methoxycarbonyl-N-(N'-methoxyacetyl-N'-2',6'-dimethylanilinomethyl)-thioharnstoff vom Fp 146 °C isoliert, die nach dem Umkristallisieren aus Isopropanol einen Fp von 148 °C aufweisen.

Folgende substituierte Thioharnstoffe der Formel I erhält man in entsprechender Weise :

| Wirkstoff Nr. | $R^1$ | $R^2$ | $R^3$ | $R^4$ | $R^5$ | $Fp(^\circ C)$ |
|---|---|---|---|---|---|---|
| 2 | $CH_3$ | $CH_3$ | H | $CO_2CH_3$ | $CH_3$ | |
| 3 | $CH_3$ | $CH_3$ | H | $CO_2CH_3$ | 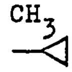 | 138 |
| 4 | $CH_3$ | $CH_3$ | H | $CO_2CH_3$ | $CH=CH_2$ | |
| 5 | $CH_3$ | $CH_3$ | H | $CO_2CH_3$ | $C=CH_2$<br>$CH_3$ | |
| 6 | $CH_3$ | $CH_3$ | H | $CO_2C_2H_5$ | $CH_2OCH_3$ | |
| 7 | $CH_3$ | $CH_3$ | H | $CO_2CH_3$ | $CH_2OC_2H_5$ | |
| 8 | $CH_3$ | $CH_2$ | H | $COSCH_3$ | $CH_2OCH_3$ | |
| 9 | $CH_3$ | $CH_3$ | $3-CH_3$ | $CO_2CH_3$ | $CH_2OCH_3$ | 164 |
| 10 | $CH_3$ | $CH_3$ | H | $CO_2CH_3$ | 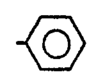 | |
| 11 | $CH_3$ | $CH_3$ | H | $CO_2CH_3$ | 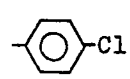 | 158 |

(Fortsetzung)

| Wirkstoff Nr. | $R^1$ | $R^2$ | $R^3$ | $R^4$ | $R^5$ | Fp(°C) |
|---|---|---|---|---|---|---|
| 12 | $CH_3$ | $CH_3$ | H | $CO_2CH_3$ | 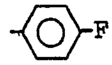 | 157 |
| 13 | $CH_3$ | $CH_3$ | H | $CO_2CH_3$ | 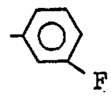 | 158 |
| 14 | $CH_3$ | $CH_3$ | H | $CO_2CH_3$ | 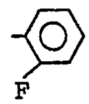 | 153 |
| 15 | $CH_3$ | $CH_3$ | H | $CO_2CH_3$ | 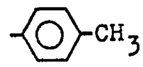 | |
| 16 | $CH_3$ | $CH_3$ | H | $CO_2CH_3$ | 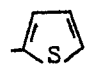 | 143 |
| 17 | $CH_3$ | $CH_3$ | H | $CO_2CH_3$ | 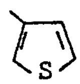 | |
| 18 | $CH_3$ | $CH_3$ | H | $CO_2CH_3$ | 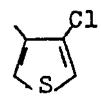 | |
| 19 | $CH_3$ | $CH_3$ | H | $CO_2CH_3$ | 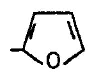 | 195 |
| 20 | $CH_3$ | $C_2H_5$ | H | $CO_2CH_3$ | 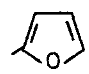 | |
| 21 | $CH_3$ | $CH_3$ | $3-CH_3$ | $CO_2CH_3$ | 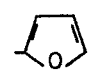 | 159 |
| 22 | $CH_3$ | $CH_3$ | H | $CO_2CH_3$ | 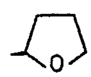 | |
| 23 | $CH_3$ | $CH_3$ | H | $CO_2CH_3$ | 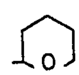 | |
| 24 | $CH_3$ | $CH_3$ | H | $CO_2CH_3$ | 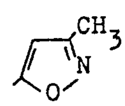 | |
| 25 | $CH_3$ | $CH_3$ | H | $CO_2CH_3$ | 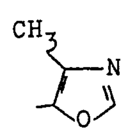 | |
| 26 | $CH_3$ | $CH_3$ | H | $CO_2CH_3$ | 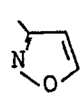 | 166 |
| 27 | $CH_3$ | $CH_3$ | H | $CO_2CH_3$ | 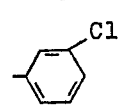 | |
| 28 | $CH_3$ | $CH_3$ | H | $CO_2CH_3$ | 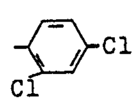 | |

Die neuen Wirkstoffe zeigen eine starke fungitoxische Wirksamkeit gegen phytopathogene Pilze, insbesondere aus der Klasse der Phycomyceten. Sie eignen sich daher beispielsweise zur Bekämpfung von Phytophthora infestans an Tomaten und Kartoffeln, Phytophthora parasitica an Erdbeeren, Phytophthora cactorum an Äpfeln, Pseudopersonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 und 5 kg Wirkstoff je ha.

Ein Teil der Wirkstoffe zeigt kurative Eigenschaften d. h. die Anwendung der Mittel kann noch nach erfolgter Infektion der Pflanzen durch die Krankheitserreger vorgenommen werden, um einen sicheren Bekämpfungserfolg zu erzielen. Darüberhinaus sind viele der neuen Verbindungen systemisch wirksam, so daß über die Wurzelbehandlung auch ein Schutz oberirdischer Pflanzenteile möglich ist.

Ferner lassen sich mit den neuen Verbindungen auch Pilze, die Keimlings- und Auflaufkrankheiten hervorrufen, beispielsweise Pythium- und Aphanomyces-Arten an Leguminosen und Baumwolle, bekämpfen. Die Aufwandmengen betragen je 100 kg Saatgut 10 bis 200 g Wirkstoff ; die Anwendung erfolgt in Form von Saatgutbeizmitteln.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekcken ; sie sollen in jedem Fall eine feine und gleichmäßige Verteilung oder wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser ; Trägerstoffe wie natürliche Gesteinsmehle, z. B. Kaoline, Tonerden, Talkum, Kreide und synthetische Gesteinsmehle (z. B. hochdisperse Kieselsäure, Silikate) ; Emulgiermittel, wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile der Verbindung des Beispiels 5 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile der Verbindung des Beispiels 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 1 werden mit 97 Gewichtsteilen feinteiligem Kaolin

innig vermischt. Man erhält auf diese Weise ein Stäubemittel, das 3 Gew.% des Wirkstoffs enthält.

VII. 30 Gewichtsteile der Verbindung des Beispiels 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäureharnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

IX. 20 Teile der Verbindung des Beispiels 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise :

Schwefel,
Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbamat oder Zinkethylenbisdithiocarbamat,
Tetramethylthiuramidsulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Propylen-bis(thiocarbamoyl)-disulfid ;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Substanzen, wie
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-pyridinmethanol,

1,2-Bis-(3-ethoxycarbonyl-2-thioureido)-benzol,
1,2-Bis-(3-methoxycarbonyl-2-thioureido)-benzol

und weitere Substanzen, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid
2,5-Dimethyl-furan-3-carbonsäureanilid,
2,5-Dimethyl-furan-3-carbonsäure-cyclohexylamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin,
1-(2-Cyano-2-phenylhexyl)-imidazol,
1-N-Propyl-N-(2-(2,4,6-trichlorphenoxy)-ethyl)-imidazol-1-carboxamid,
1-[2-(2,4-Dichlorphenyl)-4-ethyl-1,3-dioxolan-2-yl-methyl]-1H-1,2,4-triazol,
1-(2,4-Dichlorphenyl)-4,4-dimethyl-2-(1,2,4-triazol-1-yl)-pentan-3-ol.

Die folgenden Beispiele A und B zeigen die biologische Wirkung der neuen Substanzen. Als Vergleichssubstanzen dienten die bereits genannten Substanzen 2-Cyano-N-(ethylaminocarbonyl)-2-(methoxyimino)-acetamid (X) sowie N-Trichlormethylthiotetrahydrophthalimid (Y).

## Beispiel A

Fungizide Wirksamkeit gegen Phytophthora infestans an Tomaten

Blätter von Tomatenpflanzen der Sorte « Professor Rudloff » werden mit wäßrigen Suspensionen, die 80 % (Gewichtsprozent) des zu prüfenden Wirkstoffes und 20 % Natriumligninsulfonat in der Trockensubstanz enthalten, besprüht. Es werden 0,025, 0,012 und 0,006 %ige Spritzbrühen (berechnet) auf die Trockensubstanz verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

| Wirkstoff des Beispiels Nr. | Befall der Blätter nach Spritzung mit ...%iger Wirkstoffbrühe | | |
|---|---|---|---|
| | 0,025 | 0,012 | 0,006 |
| 1 | 0 | 0 | 0 |
| 14 | 1 | 2 | 2 |
| 19 | 0 | 0 | 3 |
| X | 3 | 3 | 4 |
| Kontrolle (unbehandelt) | 5 | | |

0 = kein Pilzbefall, abgestuft bis 5 = Totalbefall (Kontrolle)

Beispiel B

Fungizide Wirksamkeit gegen Auflaufkrankheiten an Erbsen

100-g-Proben Erbsensamen der Sorte « Senator » werden in Glasflaschen etwa 5 Minuten lang mit 300 mg (= 0,3 Gew.%) Beizmittelaufbereitungen, die 40 % Wirkstoff in der Trokkensubstanz enthalten, sorgfältig geschüttelt. Danach werden jeweils 100 Samen in Saatkisten 3 cm tief und mit einem Abstand von 3 bis 5 cm in eine Komposterde eingesät, die eine starke natürliche Verseuchung mit den Pilzen Pythium spec., Aphanomyces spec. und Fusarium oxysporum aufweist. Die Kästen werden im Gewächshaus bei Temperaturen von 17 bis 20 °C aufgestellt. Nach einer Versuchsdauer von 21 Tagen wird die Anzahl gesunder Erbsenpflanzen ermittelt.

| Wirkstoff des Beispiels Nr. | ... % gesunde Pflanzen nach 21 Tagen in Komposterde |
|---|---|
| 1 | 95 |
| Y | 60 |
| Kontrolle (unbehandelt) | 7 |
| Kontrolle (sterilisierte Komposterde) | 98 |

**Ansprüche**

1. Substituierte Thioharnstoffe der allgemeinen Formel

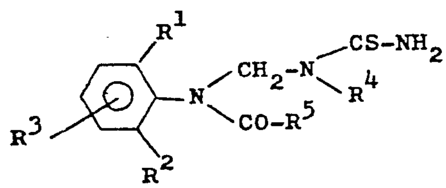

(I)

in welcher

$R^1$ $C_1$-$C_3$-Alkyl,

$R^2$ und/oder $R^3$ Wasserstoff, $C_1$-$C_3$-Alkyl oder Halogen,

$R^4$ $C_1$-$C_3$-Alkoxycarbonyl, $C_3$-$C_4$-Alkenoxycarbonyl, $C_1$-$C_2$-Alkylthiocarbonyl,

$R^5$ ein gegebenenfalls durch $C_1$-$C_3$-Alkoxy oder $C_1$-$C_3$-Alkylthio substituiertes $C_1$-$C_6$-Alkyl, ein $C_2$-$C_4$-Alkenyl, ein $C_3$-$C_6$-Cycloalkyl, einen gegebenenfalls durch Methyl oder Halogen substituierten heterocyclischen Rest bedeutet, der ein Sauerstoffatom oder ein Schwefelatom oder ein Sauerstoffatom und ein Stickstoffatom enthält, ferner ein Phenyl oder ein durch ein bis drei gleiche oder verschiedene Substituenten aus der Gruppe $C_1$-$C_3$-Alkyl, Fluor, Chlor, Brom, Trifluormethyl substituiertes Phenyl bedeutet.

2. Thioharnstoffe, ausgewählt aus der Gruppe bestehend aus

N-Methoxycarbonyl-N-(N'-methoxyacetyl-N'-2',6'-dimethylanilinomethyl)-thioharnstoff
N-Ethoxycarbonyl-N-(N'-methoxyacetyl-N'-2',6'-dimethylanilinomethyl)-thioharnstoff
N-Methoxycarbonyl-N-(N'-methoxyacetyl-N'-2',3',6'-trimethylanilinomethyl)-thioharnstoff
N-Methoxycarbonyl-N-(N'-3-fluorbenzoyl-N'-2',6'-dimethylanilinomethyl)-thioharnstoff
N-Methoxycarbonyl-N-(N'-4-fluorbenzoyl-N'-2',6'-dimethylanilinomethyl)-thioharnstoff
N-Methoxycarbonyl-N-(N'-furo-2-yl-N'-2',6'-dimethylanilinomethyl)-thioharnstoff
N-Methoxycarbonyl-N-(2'-theno-2-yl-N'-2',6'-dimethylanilinomethyl)-thioharnstoff.

3. Verfahren zur Herstellung von substituierten Thioharnstoffen der Formel I gemäß Anspruch 1, dadurch gekennzeichnet, daß man substituierte Cyanamide der Formel

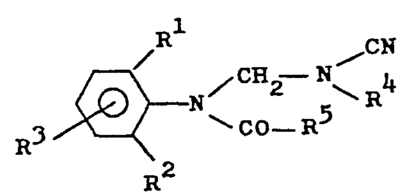

(II)

in welcher $R^1$, $R^2$, $R^3$, $R^4$ und $R^5$ die oben angegebene Bedeutung haben, bei einer Temperatur zwischen − 20 und 150 °C mit Schwefelwasserstoff umsetzt.

4. Fungizides Mittel, enthaltend eine Verbindung gemäß Anspruch 1.

5. Fungizides Mittel, enthaltend eine Verbindung gemäß Anspruch 1 und einen festen oder flüssigen Trägerstoff.

6. Verfahren zur Bekämpfung von Pilzen, dadurch gekennzeichnet, daß man mindestens eine Verbindung der Formel I gemäß Anspruch 1 auf diese einwirken läßt.

7. Verfahren zur Herstellung von Fungiziden, dadurch gekennzeichnet, daß man mindestens eine Verbindung der Formel I gemäß Anspruch 1 mit festen oder flüssigen Trägerstoffen mischt.

8. Verfahren zur vorbeugenden Bekämpfung von Pilzen, dadurch gekennzeichnet, daß man mindestens eine Verbindung der Formel I gemäß Anspruch 1 auf durch Pilzbefall bedrohte Flächen, Pflanzen oder Saatgüter einwirken läßt.

**Claims**

1. A substituted thiourea of the general formula

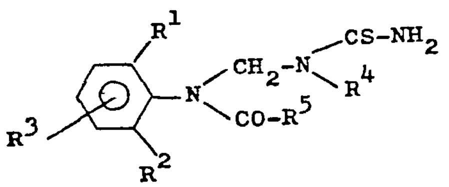

(I)

where

$R^1$ is $C_1$-$C_3$-alkyl,

$R^2$ and/or $R^3$ are hydrogen, $C_1$-$C_3$-alkyl or halogen,

$R^4$ is $C_1$-$C_3$-alkoxycarbonyl, $C_3$-$C_4$-alkenoxycarbonyl or $C_1$-$C_2$-alkylthiocarbonyl, and

$R^5$ is $C_1$-$C_6$-alkyl optionally substituted by $C_1$-$C_3$-alkoxy or $C_1$-$C_3$-alkylthio ; $C_2$-$C_4$-alkenyl ; $C_3$-$C_6$-cycloalkyl ; a heterocyclic radical optionally substituted by methyl or halogen and containing an oxygen or a sulphur atom or an oxygen and a nitrogen atom ; or phenyl optionally bearing 1 to 3 identical or different substitutents selected from the group consisting of $C_1$-$C_3$-alkyl, fluorine, chlorine, bromine and trifluoromethyl.

2. A thiourea selected from the group consisting of

N-methoxycarbonyl-N-(N'-methoxyacetyl-N'-2',6'-dimethylanilinomethyl)-thiourea,
N-ethoxycarbonyl-N-(N'-methoxyacetyl-N'-2',6'-dimethylanilinomethyl)-thiourea,
N-methoxycarbonyl-N-(N'-methoxyacetyl-N'-2',3',6'-trimethylanilinomethyl)-thiourea,
N-methoxycarbonyl-N-(N'-3-fluorobenzoyl-N'-2',6'-dimethylanilinomethyl)-thiourea,
N-methoxycarbonyl-N-(N'-4-fluorobenzoyl-N'-2',6'-dimethylanilinomethyl)-thiourea,
N-methoxycarbonyl-N-(N'-furo-2-yl-N'-2',6'-dimethylanilinomethyl)-thiourea, and
N-methoxycarbonyl-N-(2'-theno-2-yl-N'-2',6'-dimethylanilinomethyl)-thiourea.

3. A process for the preparation of a substituted thiourea of the formula I as claimed in claim 1, wherein a substituted cyanamide of the formula

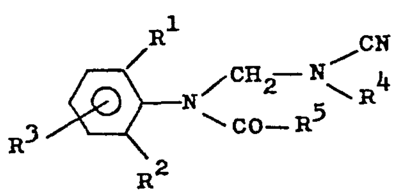

(II)

where $R^1$, $R^2$, $R^3$, $R^4$ and $R^5$ have the above meanings, is reacted with hydrogen sulfide at from − 20 to 150 °C.

4. A fungicidal agent containing a compound as claimed in claim 1.

5. A fungicidal agent containing a compound as claimed in claim 1 and a solid or liquid carrier.

6. A process for the control of fungi, wherein at least one compound of the formula I as claimed in claim 1 is allowed to act thereon.

7. A process for the production of a fungicide, wherein at least one compound of the formula I as claimed in claim 1 is mixed with a solid or liquid carrier.

8. A process for the prophylactic control of fungi, wherein at least one compound of the formula I as claimed in claim 1 is allowed to act on surfaces, plants or seeds threatened by fungal attack.

**Revendications**

1. Thiourées substituées de formule générale

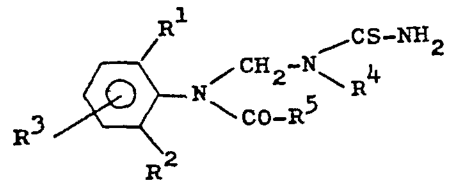

(I)

dans laquelle
$R^1$ représente alkyle en $C_1$-$C_3$
$R^2$ et/ou $R^3$, hydrogène, alkyle en $C_1$-$C_3$ ou halogène,
$R^4$ alcoxy carbonyle en $C_1$-$C_3$, alcénoxycarbonyle en $C_3$-$C_4$, alkylthiocarbonyle en $C_1$-$C_2$
$R^5$ un alkyle en $C_1$-$C_6$ éventuellement substitué par alcoxy en $C_1$-$C_3$ ou alkylthio en $C_1$-$C_3$, un alcényle en $C_2$-$C_4$, un cycloalkyle en $C_3$-$C_6$, un reste hétérocyclique, éventuellement substitué par méthyle ou halogène, et qui contient un atome d'oxygène ou un atome de soufre ou un atome d'oxygène et un atome d'azote, et représente encore un phényle ou un phényle substitué par un à trois substituants, identiques ou différents, appartenant au groupe alkyle en $C_1$-$C_3$, fluor, chlore, brome et trifluorométhyle.

2. Thiourées, choisies dans le groupe constitué par :

N-méthoxycarbonyl-N-(N'-méthoxyacétyl-N'-2',6'-di-méthylanilinométhyl)-thiourée
N-éthoxycarbonyl-N-(N'-méthoxyacétyl-N'-2',6'-di-méthylanilinométhyl)-thiourée
N-méthoxycarbonyl-N-(N'-méthoxyacétyl-N'-2',3',6'-triméthylanilinométhyl)-thiourée
N-méthoxycarbonyl-N-(N'-3-fluorobenzoyl-N'-2',6'-diméthylanilinométhyl)-thiourée
N-méthoxycarbonyl-N-(N'-4-fluorobenzoyl-N'-2',6'-diméthylanilinométhyl)-thiourée
N-méthoxycarbonyl-N-(N'-furo-2-yl-N'-2',6'-diméthylanilinométhyl)-thiourée
N-méthoxycarbonyl-N-(2'-theno-2-yl-N'-2',6'-diméthylanilinométhyl)-thiourée.

3. Procédé de préparation de thiourées substituées, de formule I, selon la revendication 1, caractérisé par le fait qu'on fait réagir des cyanamides substitués de formule

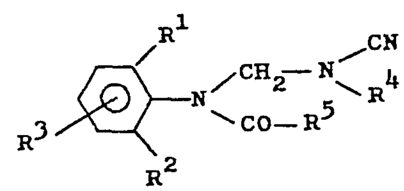

(II)

dans laquelle $R^1$, $R^2$, $R^3$, $R^4$ et $R^5$ ont les significations indiquées plus haut, à une température comprise entre − 20 et 150 °C, avec de l'hydrogène sulfuré.

4. Fongicide contenant un composé selon la revendication 1.

5. Fongicide contenant un composé selon la revendication 1 et un support solide ou liquide.

6. Procédé de lutte contre les champignons, caractérisé par le fait qu'on fait agir sur ceux-ci au moins un composé de formule I selon la revendication 1.

7. Procédé de préparation de fongicides, caractérisé par le fait qu'on mélange au moins un composé de formule I selon la revendication 1 avec des supports solides ou liquides.

8. Procédé de lutte préventive contre les champignons, caractérisé par le fait qu'on fait agir au moins un composé de formule I selon la revendication 1 sur des surfaces, plantes ou leurs graines, menacées d'une attaque par les champignons.